# EUROPEAN PATENT APPLICATION

(11) **EP 2 960 227 A1**
(43) Date of publication of application: **30.12.2015**
(21) Application number: 14754124.7
(22) Date of filing: 21.02.2014
(51) Int. Cl.: C07C 51/41, C07C 61/09, C07C 63/28, C07F 1/08

(54) **METHOD FOR PRODUCING METAL COMPLEX**

(30) Priority: 22.02.2013 JP 2013033263
(71) Applicant: Kuraray Co., Ltd., Kurashiki-shi, Okayama 710-0801 (JP)
(72) Inventor: INUBUSHI, Yasutaka, Kurashiki-shi Okayama 710-0801 (JP); MIYAUCHI, Chikako, Kurashiki-shi Okayama 710-0801 (JP); TORIHARA, Masahiro, Kurashiki-shi Okayama 710-0801 (JP)
(74) Representative: Müller-Boré & Partner Patentanwälte PartG mbB
(86) International application number: PCT/JP2014/054112
(87) International publication number: WO 2014/129569

(57) **Abstract**

Disclosed is a method for producing a metal complex comprising a multivalent carboxylic acid compound, at least one metal ion, an organic ligand capable of multidentate binding to the metal ion, and a monocarboxylic acid compound, wherein the metal ion is used in the form of a metal salt having a counter anion of the metal ion, a conjugate acid of the counter anion having a first dissociation exponent larger by 0 to 6 than that of the multivalent carboxylic acid compound, and at least one of the multivalent carboxylic acid compound, the metal ion, the organic ligand capable of multidentate binding, and the monocarboxylic acid compound is reacted in a suspended state. This method can effectively produce a metal complex.

## Description

### Technical Field

### Cross-Reference to Related Application

This application claims priority to Japanese Patent Application No. 2013-033263 filed on February 22, 2013, the disclosure of which is incorporated herein by reference in its entirety.

### Technical Field

The present invention relates to a method for producing a metal complex. More specifically, the present invention relates to a method for producing a metal complex comprising a multivalent carboxylic acid compound, at least one metal ion selected from ions of metals belonging to Groups 2 to 13 of the periodic table, an organic ligand capable of multidentate binding to the metal ion, and a monocarboxylic acid compound.

### Background Art

In the fields of deodorization, exhaust gas treatment, and the like, various adsorbent materials have so far been developed. Activated carbon is a representative example of these, and has been widely used in various industries for the purpose of air cleaning, desulfurization, denitrification, or removal of harmful substances by making use of its excellent adsorption performance. In recent years, demand for nitrogen has been increasing, for example, in semiconductor manufacturing processes and the like. Such nitrogen is produced from air by using molecular sieving carbon according to the pressure swing adsorption process or temperature swing adsorption process. Molecular sieving carbon is also used for separation and purification of various gases, such as purification of hydrogen from cracked methanol gas.

When a mixture of gases is separated according to the pressure swing adsorption process or temperature swing adsorption process, it is common practice to separate it based on the difference between the gases in equilibrium adsorption amount or rate of adsorption to molecular sieving carbon or zeolite used as a separation adsorbent material. When the mixture of gases is separated based on the difference in equilibrium adsorption amount, conventional adsorbent materials cannot selectively adsorb only the gas to be removed, and the separation coefficient decreases, inevitably increasing the size of the apparatus used. When the mixture of gases is separated into individual gases based on the difference in rate of adsorption, on the other hand, only the gas to be removed can be adsorbed, although it depends on the kind of gas. It is necessary, however, to alternately perform adsorption and desorption; in this case, as well, the apparatus used should be larger.

On the other hand, a polymer metal complex has also been developed as an adsorbent material providing superior adsorption performance. The polymer metal complex has features including (1) a large surface area and high porosity, (2) high designability, and (3) a change in dynamic structure when exposed to external stimulation. The polymer metal complex is expected to attain adsorption properties that known adsorbent materials do not have.

As a metal complex having excellent adsorption performance, Patent Literature (PTL) 1 discloses a polymer metal complex comprising a copper ion, terephthalic acid, and 4,4'-bipyridyl.

Further, Non-patent Literature (NPL) 1 discloses a polymer metal complex comprising a copper ion, terephthalic acid, and 4,4'-bipyridyl.

### Citation List

### Patent Literature

PTL 1: JP2003-342260A

### Non-patent Literature

NPL 1: Yoko Sakata, Shuhei Furukawa, Mio Kondo, Kenji Hirai, Nao Horike, Yohei Takashima, Hiromitsu Uehara, Nicolas Louvain, Mikhail Meilikhov, Takaaki Tsuruoka, Seiji Isoda, Wataru Kosaka, Osami Sakata, Susumu Kitagawa, Science, Vol. 339, pp. 193-196 (2013)

### Summary of Invention

### Technical Problem

However, the results of confirmatory tests by the present inventors revealed that the methods for producing metal complexes disclosed in PTL 1 and NPL 1 had problematic productivity. It was also revealed that the metal complex of NPL 1 had a small average particle diameter, and problematic adsorption performance.

Therefore, an object of the present invention is to provide a method for efficiently producing a metal complex.

### Solution to Problem

As a result of intensive study, the present inventors found that the above object can be achieved by a method for producing a metal complex comprising a multivalent carboxylic acid compound, at least one metal ion, an organic ligand capable of multidentate binding to the metal ion, and a monocarboxylic acid compound, wherein the metal ion is used in the form of a metal salt having a counter anion of the metal ion, in which a value obtained by subtracting the first dissociation exponent of the multivalent carboxylic acid compound from the first dissociation exponent of a conjugate acid of the counter anion is within the range of 0 to 6, and at least one of the multivalent carboxylic acid, the metal ion, the organic ligand capable of multidentate binding, and the monocarboxylic acid compound is reacted in a suspended state. The present invention has thus been accomplished.

Specifically, the present invention provides the following:
(1) A method for producing a metal complex comprising a multivalent carboxylic acid compound, at least one metal ion selected from ions of metals belonging to Groups 2 to 13 of the periodic table, an organic ligand capable of multidentate binding to the metal ion, and a monocarboxylic acid compound;
   the method comprising the step of reacting the multivalent carboxylic acid compound, the at least one metal ion selected from ions of metals belonging to Groups 2 to 13 of the periodic table, the organic ligand capable of multidentate binding to the metal ion, and the monocarboxylic acid compound in a single stage or multiple stages,
   wherein in the reacting step, the metal ion is used in the form of a metal salt having a counter anion of the metal ion, a conjugate acid of the counter anion having a first dissociation exponent larger by 0 to 6 than that of the multivalent carboxylic acid compound, and
   at least one of the multivalent carboxylic acid, the metal ion, the organic ligand capable of multidentate binding, and the monocarboxylic acid compound is reacted in a suspended state.
(2) The method for producing a metal complex according to (1), wherein the reacting step is performed in a solvent.
(3) The method for producing a metal complex according to (1) or (2), wherein the reacting step comprises:
   a first step of reacting the multivalent carboxylic acid compound, the metal ion, and the monocarboxylic acid compound; and
   a second step of reacting a product obtained in the first step with the organic ligand capable of multidentate binding.
(4) The method for producing a metal complex according to (3), wherein the first step is performed at a reaction temperature of 303 to 373 K.
(5) The method for producing a metal complex according to any one of (1) to (4), wherein the counter anion of the metal ion is an aliphatic monocarboxylate ion.
(6) The method for producing a metal complex according to any one of (1) to (5), wherein the multivalent carboxylic acid compound is a dicarboxylic acid compound.
(7) The method for producing a metal complex according to any one of (1) to (6), wherein the organic ligand capable of multidentate binding is an organic ligand capable of bidentate binding.
(8) The method for producing a metal complex according to (7), wherein the organic ligand capable of bidentate binding belongs to the D_{∞h} point group and has a longitudinal length of 7.0 Å or more and 16.0 Å or less.
(9) The method for producing a metal complex according to any one of (1) to (8), wherein the metal complex obtained by the method for producing a metal complex has an average particle diameter of 0.1 to 10 µm.
(10) A metal complex obtained by the production method according to any one of (1) to (9).

### Advantageous Effects of Invention

The present invention provides a method for producing a metal complex comprising a multivalent carboxylic acid compound, at least one metal ion, an organic ligand capable of multidentate binding to the metal ion, and a monocarboxylic acid compound.

### Brief Description of Drawings

Fig. 1 shows a schematic diagram illustrating a jungle-gym-type framework in which 4,4'-bipyridyl is coordinated to the axial position of a metal ion in a paddle-wheel-type framework composed of a copper ion and a carboxylate group of terephthalic acid.
Fig. 2 shows a schematic diagram illustrating a three-dimensional structure in which two jungle-gym-type frameworks are interpenetrated into each other.
Fig. 3 shows a powder X-ray diffraction pattern of a metal complex obtained in Example 1.
Fig. 4 shows the crystal structure of the metal complex obtained in Example 1.
Fig. 5 shows a ¹H-NMR spectrum measured by dissolving the metal complex obtained in Example 1 in deuterated ammonia water.
Fig. 6 shows the particle size distribution of the metal complex obtained in Example 1.
Fig. 7 shows adsorption and desorption isotherms of carbon dioxide on the metal complex obtained in Example 1 at 293 K.
Fig. 8 shows a powder X-ray diffraction pattern of a metal complex obtained in Example 2.
Fig. 9 shows a ¹H-NMR spectrum measured by dissolving the metal complex obtained in Example 2 in deuterated ammonia water.
Fig. 10 shows a ¹H-NMR spectrum measured by dissolving the metal complex obtained in Example 2 in a mixed solution of deuterated ammonia water and deuterated trifluoroacetic acid.
Fig. 11 shows adsorption and desorption isotherms of carbon dioxide on the metal complex obtained in Example 2 at 293 K.
Fig. 12 shows a powder X-ray diffraction pattern of a metal complex obtained in Example 3.
Fig. 13 shows adsorption and desorption isotherms of carbon dioxide on the metal complex obtained in Example 3 at 293 K.
Fig. 14 shows a powder X-ray diffraction pattern of a metal complex obtained in Comparative Example 1.
Fig. 15 shows adsorption and desorption isotherms of carbon dioxide on the metal complex obtained in Comparative Example 1 at 293 K.
Fig. 16 shows a powder X-ray diffraction pattern of a metal complex obtained in Comparative Example 2.
Fig. 17 shows adsorption and desorption isotherms of carbon dioxide on the metal complex obtained in Comparative Example 2 at 293 K.
Fig. 18 shows a powder X-ray diffraction pattern of a metal complex obtained in Comparative Example 3.
Fig. 19 shows adsorption and desorption isotherms of carbon dioxide on the metal complex obtained in Comparative Example 3 at 293 K.
Fig. 20 shows a powder X-ray diffraction pattern of a metal complex obtained in Example 4.
Fig. 21 shows the crystal structure of the metal complex obtained in Example 4.
Fig. 22 shows a ¹H-NMR spectrum measured by dissolving the metal complex obtained in Example 4 in deuterated ammonia water.
Fig. 23 shows adsorption and desorption isotherms of methane on the metal complex obtained in Example 4 at 298 K.
Fig. 24 shows a powder X-ray diffraction pattern of a metal complex obtained in Comparative Example 5.

In the measurement results of a powder X-ray diffraction pattern, the horizontal axis represents a diffraction angle (2θ), and the vertical axis represents a diffraction intensity expressed in cps (counts per second).

In the measurement results of adsorption and desorption isotherms, the horizontal axis represents an equilibrium pressure expressed in MPa, and the vertical axis represents an equilibrium amount adsorbed expressed in mL(STP)/g. In the measurement results of adsorption and desorption isotherms, the amount of gas (e.g., carbon dioxide or methane) adsorbed (ads.) under increased pressure and the amount of gas desorbed (des.) under decreased pressure are plotted for each pressure level. "STP" (standard temperature and pressure) denotes a state at a temperature of 273.15 K and a pressure of 1 bar (10⁵ Pa).

### Description of Embodiments

The present invention relates to a method for producing a metal complex comprising a multivalent carboxylic acid compound, at least one metal ion, an organic ligand capable of multidentate binding to the metal ion, and a monocarboxylic acid compound.

The multivalent carboxylic acid compound used in the present invention is not particularly limited. Dicarboxylic acid compounds, tricarboxylic acid compounds, tetracarboxylic acid compounds, and the like, can be used.

Examples of the multivalent carboxylic acid compound used in the present invention include saturated aliphatic dicarboxylic acids, such as succinic acid, adipic acid, and trans-1,4-cyclohexanedicarboxylic acid; unsaturated aliphatic dicarboxylic acids, such as fumaric acid and trans,trans-1,4-butadienedicarboxylic acid; aromatic dicarboxylic acids, such as isophthalic acid, terephthalic acid, 1,4-naphthalenedicarboxylic acid, 2,6-naphthalenedicarboxylic acid, 2,7-naphthalenedicarboxylic acid, and 4,4'-biphenyldicarboxylic acid; heteroaromatic dicarboxylic acids, such as 2,5-thiophenedicarboxylic acid, 2,2'-dithiophenedicarboxylic acid, 2,3-pyrazinedicarboxylic acid, 2,5-pyridinedicarboxylic acid, and 3,5-pyridinedicarboxylic acid; aromatic tricarboxylic acids, such as 1,3,5-benzenetricarboxylic acid, 1,3,4-benzenetricarboxylic acid, and biphenyl-3,4',5-tricarboxylic acid; aromatic tetracarboxylic acids, such as 1,2,4,5-benzenetetracarboxylic acid, [1,1':4',1"]terphenyl-3,3",5,5"-tetracarboxylic acid, and 5,5'-(9,10-anthracenediyl)diisophthalate; and the like. Among these, dicarboxylic acid compounds are preferable; and aromatic dicarboxylic acid compounds are more preferable.

The multivalent carboxylic acid compound may further comprise a substituent other than carboxyl. The multivalent carboxylic acid having a substituent is preferably an aromatic multivalent carboxylic acid, and a substituent preferably binds to the aromatic ring of the aromatic multivalent carboxylic acid. The number of substituents is 1, 2, or 3. Examples of substituents include, but are not particularly limited to, alkyl groups (linear or branched alkyl groups having 1 to 5 carbon atoms, such as methyl, ethyl, *n*-propyl, isopropyl, *n*-butyl, isobutyl, *tert*-butyl, and pentyl), halogen atoms (fluorine, chlorine, bromine, and iodine), alkoxy groups (e.g., methoxy, ethoxy, *n*-propoxy, isopropoxy, *n*-butoxy, isobutoxy, and *tert-*butoxy), amino groups, monoalkylamino groups (e.g., methylamino), dialkylamino groups (e.g., dimethylamino), formyl groups, epoxy groups, acyloxy groups (e.g., acetoxy, *n*-propanoyloxy, n-butanoyloxy, pivaloyloxy, and benzoyloxy), alkoxycarbonyl groups (e.g., methoxycarbonyl, ethoxycarbonyl, and *n*-butoxycarbonyl), nitro groups, cyano groups, hydroxyl groups, acetyl groups, trifluoromethyl groups, and the like. Specific examples include multivalent carboxylic acid compounds having a substituent such as 2-nitroterephthalic acid, 2-fluoroterephthalic acid, 1,2,3,4-tetrafluoroterephthalic acid, 2,4,6-trifluoro-1,3,5-benzenetricarboxylic acid, or the like.

The multivalent carboxylic acid compounds may be used singly or in a mixture of two or more. The metal complex produced by the production method of the present invention may be a mixture of two or more metal complexes each containing a single multivalent carboxylic acid compound.

The multivalent carboxylic acid compound may be used in the form of an acid anhydride or an alkali metal salt.

The metal ion used in the present invention is at least one metal ion selected from ions of metals belonging to Groups 2 to 13 of the periodic table. The ions of metals belonging to Group 2 of the periodic table include beryllium, magnesium, calcium, strontium, barium, and radium ions. The ions of metals belonging to Group 3 of the periodic table include scandium, yttrium, lanthanide, and actinoid ions. The ions of metals belonging to Group 4 of the periodic table include titanium, zirconium, hafnium, and rutherfordium ions. The ions of metals belonging to Group 5 of the periodic table include vanadium, niobium, tantalum, and dubnium ions. The ions of metals belonging to Group 6 of the periodic table include chromium, molybdenum, tungsten, and seaborgium ions. The ions of metals belonging to Group 7 of the periodic table include manganese, technetium, rhenium, and bohrium ions. The ions of metals belonging to Group 8 of the periodic table include iron, ruthenium, osmium, and hassium ions. The ions of metals belonging to Group 9 of the periodic table include cobalt, rhodium, iridium, and meitnerium ions. The ions of metals belonging to Group 10 of the periodic table include nickel, palladium, platinum, and darmstadtium ions. The ions of metals belonging to Group 11 of the periodic table include copper, silver, gold, and roentgenium ions. The ions of metals belonging to Group 12 of the periodic table include zinc, cadmium, mercury, and ununbium ions. The ions of metals belonging to Group 13 of the periodic table include boron, aluminum, gallium, indium, thallium, and ununtrium ions.

Examples of ions of metals belonging to Groups 2 to 13 of the periodic table used in the present invention include magnesium, calcium, scandium, lanthanide (e.g., lantern, terbium, and lutetium), actinoid (e.g., actinium and lawrencium), zirconium, vanadium, chromium, molybdenum, manganese, iron, cobalt, nickel, copper, zinc, cadmium, aluminum, and like ions. Among these, manganese, cobalt, nickel, copper, and zinc ions are preferable; and copper ions are more preferable.

The metal ion used in the present invention may be a single metal ion or a mixture of two or more metal ions. The metal complex obtained by the production method of the present invention may be a mixture of two or more metal complexes each containing a single metal ion.

In the production method of the present invention, the metal ion is used in the form of a metal salt. Usable examples of metal salts include magnesium salts, calcium salts, scandium salts, lanthanide salts (e.g., lantern salt, terbium salt, and lutetium salt), actinoid salts (e.g., actinium salt and lawrencium salt), zirconium salts, vanadium salts, chromium salts, molybdenum salts, manganese salts, iron salts, cobalt salts, nickel salts, copper salts, zinc salts, cadmium salts, and aluminum salts. Among these, manganese salts, cobalt salts, nickel salts, copper salts, and zinc salts are preferable; and copper salts are more preferable.

The metal salt may be a single metal salt or a mixture of two or more metal salts.

In the production method of the present invention, it is necessary to use a metal salt having a counter anion of the metal ion, the conjugate acid of the counter anion having a first dissociation exponent larger by 0 to 6 than that of the multivalent carboxylic acid compound. The first dissociation exponent (pKₐₗ) of acid is the negative common logarithm of the acid dissociation constant (Kₐ, 25°C) (pKₐ₁ = -logKₐ). For example, when copper acetate is used as the metal salt, the first dissociation exponent of acetic acid, which is the conjugate acid of the acetate ion that is a counter anion, is 4.82. On the other hand, when terephthalic acid is used as the multivalent carboxylic acid compound, the first dissociation exponent of terephthalic acid is 3.51. In this case, the first dissociation exponent of the acetic acid, which is the conjugate acid of the acetate ion that is a counter anion of the metal salt, is larger by 1.31 than the first dissociation exponent of the terephthalic acid. That is, it is preferable to use a metal salt containing a conjugate acid that is as acidic as or less acidic than the multivalent carboxylic acid compound.

Examples of the metal salt used in the production method of the present invention include organic acid salts, such as acetate, formate, and terephthalate; and inorganic acid salts, such as carbonate. Among these, acetate, formate, and terephthalate are preferable; acetate or formate is more preferable; and acetate is particularly preferable. For example, the first dissociation exponent of formic acid is 3.75, the first dissociation exponent of acetic acid is 4.82, and the first dissociation exponent of terephthalic acid is 3.51. The counter anion of the metal salt may be a counter anion of a conjugate acid with a first dissociation exponent equal to that of the multivalent carboxylic-acid compound. For example, when terephthalic acid is used as the multivalent carboxylic acid compound, copper terephthalate may be used as the metal salt. In this case, the difference in the first dissociation exponent is 0. Comparatively, for example, copper sulfate is not suitable when terephthalic acid is used as the multivalent carboxylic acid compound, because the first dissociation exponent of sulfuric acid, which is a conjugate acid, is -5.00.

The organic ligand capable of multidentate binding to the metal ion used in the present invention refers to a neutral ligand having at least two sites coordinated to the metal ion with a lone electron pair. The bidentate organic ligand refers to a neutral organic ligand having two sites coordinated to the metal ion with a lone electron pair. The tridentate organic ligand refers to a neutral organic ligand having three sites coordinated to the metal ion with a lone electron pair. The tetradentate organic ligand refers to a neutral organic ligand having four sites coordinated to the metal ion with a lone electron pair.

Examples of sites coordinated to a metal ion with a lone electron pair include nitrogen atoms, oxygen atoms, phosphorus atoms, sulfur atoms, and the like. The organic ligand capable of multidentate binding is preferably a heteroaromatic ring compound, in particular, a heteroaromatic ring compound that has nitrogen atoms as coordination sites. The heteroaromatic ring compound may be substituted, or may be bound together by a divalent hydrocarbon group (e.g., a divalent group obtained by removing two hydrogen atoms from ethyne).

Examples of organic ligands capable of bidentate binding (bidentate ligands) include 1,4-diazabicyclo[2.2.2]octane, pyrazine, 4,4'-bipyridyl, 1,2-bis(4-pyridyl)ethyne, 1,4-bis(4-pyridyl)butadiyne, 1,4-bis(4-pyridyl)benzene, 3,6-di(4-pyridyl)-1,2,4,5-tetrazine, 2,2'-bi-1,6-naphthyridine, phenazine, diazapyrene, 2,6-di(4-pyridyl)-benzo[1,2-c:4,5-c']dipyrrole-1,3,5,7(2H,6H)-tetron, N,N'-di(4-pyridyl)-1,4,5,8-naphthalenetetracarboxydiimide, trans-1,2-bis(4-pyridyl)ethene, 4,4'-azopyridine, 1,2-bis(4-pyridyl)ethane, 4,4'-dipyridyl sulfide, 1,3-bis(4-pyridyl)propane, 1,2-bis(4-pyridyl)-glycol, N-(4-pyridyl)isonicotinamide, 1,2-bis(1-imidazolyl)ethane, 1,2-bis(1,2,4-triazolyl)ethane, 1,2-bis(1,2,3,4-tetrazolyl)ethane, 1,3-bis(1-imidazolyl)propane, 1,3-bis(1,2,4-trizolyl)propane, 1,3-bis(1,2,3,4-tetrazolyl)propane, 1,4-bis(4-pyridyl)butane, 1,4-bis(1-imidazolyl)butane, 1,4-bis(1,2,4-triazolyl)butane, 1,4-bis(1,2,3,4-tetrazolyl)butane, 1,4-bis(benzimidazole-1-ylmethyl)-2,4,5,6-tetramethyl benzene, 1,4-bis(4-pyridylmethyl)-2,3,5,6-tetramethyl benzene, 1,3-bis(imidazole-1-ylmethyl)-2,4,6-trimethyl benzene, 1,3-bis(4-pyridylmethyl)-2,4,6-trimethyl benzene, and the like. Examples of organic ligands capable of tridentate binding (tridentate ligands) include 1,3,5-tris(2-pyridyl)benzene, 1,3,5-tris(3-pyridyl)benzene, 1,3,5-tris(4-pyridyl)benzene, 1,3,5-tris(1-imidazolyl)benzene, 2,4,6-tris(2-pyridyl)-1,3,5-triazine, 2,4,6-tris(3-pyridyl)-1,3,5-triazine, 2,4,6-tris(4-pyridyl)-1,3,5-triazine, 2,4,6-tris(1-imidazolyl)-1,3,5-triazine, and the like. Examples of organic ligands capable of tetradentate binding (tetradentate ligands) include 1,2,4,5-tetrakis(2-pyridyl)benzene, 1,2,4,5-tetrakis(3-pyridyl)benzene, 1,2,4,5-tetrakis(4-pyridyl)benzene, 1,2,4,5-tetrakis(1-imidazolyl)benzene, tetrakis(3-pyridyloxymethylene)methane, tetrakis(4-pyridyloxymethylene)methane, tetrakis(1-imidazolylmethyl)methane, and the like. Among these, organic ligands capable of bidentate binding are preferable.

The organic ligand capable of multidentate binding may have a substituent. Although the substituent is not particularly limited, examples thereof include one or more (preferably 1 to 3) alkyl groups having 1 to 5 carbon atoms. Examples of the organic ligand capable of multidentate binding having a substituent include 2-methylpyrazine, 2,5-dimethylpyrazine, 2,2'-dimethyl-4,4'-bipyridine, and the like.

The organic ligands capable of multidentate binding may be used singly or in a mixture of two or more. The metal complex obtained by the production method of the present invention may be a mixture of two or more metal complexes each containing a single organic ligand capable of multidentate binding.

Of the organic ligands capable of multidentate binding used in the present invention, an organic ligand capable of bidentate binding that belongs to the D_{∞h} point group and has a longitudinal length of 7.0 Å or more and 16.0 Å or less is preferable. The point group to which the organic ligand capable of bidentate binding belongs may be determined according to the method disclosed in Reference Document 1 below.

Reference Document 1: Bunshino Taisho to Gunron, Molecular Symmetry and Group Theory; Masao Nakazaki, 1973, Tokyo Kagaku Dojin Co., Ltd., pp. 39-40.

For example, since 4,4'-bipyridyl, 1,2-bis(4-pyridyl)ethyne, 2,7-diazapyrene, 1,4-bis(4-pyridyl)benzene, 1,4-bis(4-pyridyl)butadiyne, 3,6-di(4-pyridyl)-1,2,4,5-tetrazine, 2,6-di(4-pyridyl)-benzo[1,2-c:4,5-c']dipyrrole-1,3,5,7(2H,6H)-tetron, 4,4'-bis(4-pyridyl)biphenyl, *N,N'*-di(4-pyridyl)-1,4,5,8-naphthalenetetracarboxydiimide, and the like are bilaterally symmetric linear molecules having a symmetric center, they belong to the D_{∞h} point group. Further, since 1,2-bis(4-pyridyl)ethene has a two-fold axis and symmetric planes perpendicular to the axis, it belongs to the C₂ₕ point group.

When the point group of the organic ligand capable of bidentate binding is D_{∞h}, the high symmetry reduces wasteful gaps. Thus, high adsorption performance can be exhibited. In addition, when the longitudinal length of the organic ligand capable of bidentate binding is 7.0 Å or more and 16.0 Å or less, the distance between the metal ions in the metal complex will be suitable. Thus, a metal complex having optimal gaps for adsorbing and desorbing a gas molecule can be formed. A metal complex can be obtained by using an organic ligand capable of bidentate binding that has a longitudinal length outside of the above range; however, storage performance and separation performance tend to decrease.

The longitudinal length of the organic ligand capable of bidentate binding in the present specification is defined as the distance between two atoms having the longest distance between them, among the atoms coordinated to the metal ion in the structural formula, in the most stable structure found by structure optimization according to the PM5 semiempirical molecular orbital method after the conformational analysis according to the MM3 molecular dynamics method using Scigress Explorer Professional, Version 7.6.0.52 (produced by Fujitsu).

For example, the distance between nitrogen atoms of 1,4-diazabicyclo[2.2.2]octane is 2.609 Å, the distance between nitrogen atoms of pyrazine is 2.810 Å, the distance between nitrogen atoms of 4,4'-bipyridyl is 7.061 Å, the distance between nitrogen atoms of 1,2-bis(4-pyridyl)ethyne is 9.583 Å, the distance between nitrogen atoms of 1,4-bis(4-pyridyl)benzene is 11.315 Å, the distance between nitrogen atoms of 3,6-di(4-pyridyl)-1,2,4,5-tetrazine is 11.204 Å, the distance between nitrogen atoms of 2,6-di(4-pyridyl)-benzo[1,2-c:4,5-c']dipyrrole-1,3,5,7(2H,6H)-tetron is 15.309 Å, the distance between nitrogen atoms of 4,4'-bis(4-pyridyl)biphenyl is 15.570 Å, and the distance between nitrogen atoms of *N,N'-*di(4-pyridyl)=1,4,5,8-naphthalenetetracarboxydiimide is 15.533 Å.

Preferable examples of the organic ligand capable of multidentate binding used in the production method of the present invention include 4,4'-bipyridyl, 2,7-diazapyrene, 1,2-bis(4-pyridyl)ethyne, 1,4-bis(4-pyridyl)butadiyne, 1,4-bis(4-pyridyl)benzene, 3,6-di(4-pyridyl)-1,2,4,5-tetrazine, 2,6-di(4-pyridyl)-benzo[1,2-c:4,5-c']dipyrrole-1,3,5,7(2H,6H)-tetron, 4,4'-bis(4-pyridyl)biphenyl, *N,N'*-di(4-pyridyl)-1,4,5,8-naphthalenetetracarboxydiimide, and the like. Among these, 4,4'-bipyridyl is particularly preferable.

Examples of the monocarboxylic acid compound used in the production method of the present invention include formic acid; aliphatic monocarboxylic acids, such as acetic acid, propionic acid, butyric acid, isobutyric acid, valeric acid, caproic acid, enanthic acid, cyclohexanecarboxylic acid, caprylic acid, octylic acid, pelargonic acid, capric acid, lauric acid, myristic acid, pentadecylic acid, palmitic acid, margaric acid, stearic acid, tuberculostearic acid, arachidic acid, behenic acid, lignoceric acid, α-linolenic acid, eicosapentaenoic acid, docosahexaenoic acid, linoleic acid, and oleic acid; aromatic monocarboxylic acids, such as benzoic acid; heteroaromatic monocarboxylic acids, such as nicotinic acid and isonicotinic acid; and the like. Among these, formic acid, acetic acid, octylic acid, lauric acid, myristic acid, palmitic acid, and stearic acid are preferable; particularly, formic acid or acetic acid is more preferable.

The monocarboxylic acid compounds may be used singly or in a mixture of two or more. Moreover, the metal complex of the present invention may be a mixture of two or more metal complexes each containing a single monocarboxylic acid compound.

The monocarboxylic acid compound may be used in the form of an acid anhydride or an alkali metal salt in the manufacture of the metal complex. The monocarboxylic acid compound can be incorporated in the metal complex structure of the present invention when used as a counter anion of the raw material metal salt. For example, when a copper ion and acetic acid are respectively used as the metal ion and the monocarboxylic acid compound, they may be used in the form of copper acetate.

In the production method of the present invention, the target metal complex can be obtained by reacting a multivalent carboxylic acid compound, at least one metal ion selected from ions of metals belonging to Groups 2 to 13 of the periodic table, an organic ligand capable of multidentate binding to the metal ion, and a monocarboxylic acid compound. The step of "reacting a multivalent carboxylic acid compound, at least one metal ion selected from ions of metals belonging to Groups 2 to 13 of the periodic table, an organic ligand capable of multidentate binding to the metal ion, and a monocarboxylic acid compound" includes reacting a multivalent carboxylic acid compound, at least one metal ion selected from ions of metals belonging to Groups 2 to 13 of the periodic table, an organic ligand capable of multidentate binding to the metal ion, and a monocarboxylic acid compound in a single stage or multiple stages. That is, this step includes reacting these four components in a single stage by simultaneously mixing them, and reacting them in multiple stages by mixing at least one of these four components with other components at different timing.

The method for producing a metal complex according to the present invention preferably comprises the steps of:
(i) reacting a multivalent carboxylic acid compound and at least one metal ion selected from ions of metals belonging to Groups 2 to 13 of the periodic table;
(ii) reacting the resultant with a monocarboxylic acid compound to obtain an intermediate; and
(iii) reacting the intermediate with an organic ligand capable of multidentate binding to the metal ion to obtain a metal complex.
Step (ii) may be performed simultaneously with or after step (i). Step (iii) may be performed simultaneously with or after steps (i) and (ii). Alternatively, step (ii) may be performed after step (i), and step (iii) may be further performed after step (ii). It is preferable to perform these steps in a solvent.

The solvent may be an organic solvent, water, or a mixed solvent of these. Specific examples of solvents include methanol, ethanol, propanol, diethylether, butanol, dimethoxyethane, tetrahydrofuran, hexane, cyclohexane, heptane, benzene, toluene, methylene chloride, chloroform, acetone, ethyl acetate, acetonitrile, N,N-dimethylformamide, water, and mixed solvents of these substances. The metal complex is preferably produced by reacting these four components in a solvent under ordinary pressure for several hours to several days, and precipitating the metal complex. The reaction may be performed under ultrasonic or microwave irradiation.

In a preferred embodiment, the production method of the present invention is divided into two steps: a first step of reacting a multivalent carboxylic acid compound, a metal ion, and a monocarboxylic acid compound, and a second step of reacting a intermediate obtained in the first step with an organic ligand capable of multidentate binding to the metal ion, thereby obtaining a metal complex. In the first step, a solution in which the multivalent carboxylic acid compound is dispersed may be sequentially mixed with a solution in which the metal ion is dispersed; the opposite is also possible. In that case, the monocarboxylic acid compound may be added to either of the solutions. Further, in the second step, a solution in which the intermediate is dispersed may be sequentially mixed with a solution in which the organic ligand capable of multidentate binding is dispersed; the opposite is also possible.

Moreover, the method for producing a metal complex according to the present invention characteristically comprises the step of reacting at least one of a multivalent carboxylic acid compound, a metal ion, an organic ligand capable of multidentate binding to the metal ion, and a monocarboxylic acid compound in a suspended state. The suspended state indicates that at least one of the four components is added to the reaction system so that the concentration of at least one of the four components in the reaction system after addition is equal to or greater than its saturated solubility, and particles are dispersed in the liquid. It is preferable that the reaction is performed in a suspended state in the step of reacting a multivalent carboxylic acid compound and a metal ion, or in the step of reacting a multivalent carboxylic acid compound, a metal ion, and a monocarboxylic acid compound. Among the four components, it is preferable to react a multivalent carboxylic acid compound in a suspended state, and it is more preferable to react a multivalent carboxylic acid compound and an organic ligand capable of multidentate binding in a suspended state. The reaction in a suspended state can increase volume efficiency, thus improving the productivity of the metal complex.

When a multivalent carboxylic acid compound and a metal ion are reacted, the multivalent carboxylic acid compound is deprotonated and coordinated to the metal ion. Along with the progress of the reaction, the conjugate acid of the counter anion of the metal salt is produced as a by-product and accumulated in the reaction system. Therefore, when the first dissociation exponent of the conjugate acid of the counter anion of the metal ion used is smaller than that of the multivalent carboxylic acid compound used, the deprotonation of the multivalent carboxylic acid compound is inhibited, thereby stopping the reaction halfway. Furthermore, the conjugate acid produced as a by-product increases the acidity of the reaction system, and promotes side reactions. This may cause more serious problems when the reaction is performed in a suspended state of a high concentration of a component. Therefore, in the production method of the present invention, it is important to use a metal salt in which the first dissociation exponent of the conjugate acid of the counter anion of the metal ion is larger by 0 to 6 than the first dissociation exponent of the multivalent carboxylic acid compound.

The completion of the reaction may be confirmed by analyzing the remaining amount of the raw materials by the Pack Test using absorption spectrophotometry, or by gas chromatography or high-performance liquid chromatography; however, the method is not limited to them. After the reaction is completed, the resulting mixture is subjected to suction filtration to collect the precipitate. The precipitate is washed with an organic solvent and dried in vacuum for several hours at about 373 K, thereby obtaining the metal complex of the present invention.

The reaction temperature can be suitably selected according to the solvent used, and it is preferably 303 to 373 K, and more preferably 303 to 353 K. When the reaction is performed in multiple stages, the reaction temperature is preferably 303 to 373 K, and more preferably 303 to 353 K, at least in the first step of reacting a multivalent carboxylic acid compound, a metal ion, and a monocarboxylic acid compound.

The mixing ratio of the metal salt to the multivalent carboxylic acid compound during the manufacture of the metal complex is preferably such that the molar ratio of the metal salt to the multivalent carboxylic acid compound is 1:5 to 8:1, and more preferably 1:3 to 6:1. The mixing ratio of the metal salt to the organic ligand capable of multidentate binding is preferably such that the molar ratio of the metal salt to the organic ligand capable of multidentate binding is 1:3 to 3:1, and more preferably 1:2 to 2:1. The mixing ratio of the multivalent carboxylic acid compound to the monocarboxylic acid compound is preferably such that the molar ratio of the multivalent carboxylic acid compound to the monocarboxylic acid compound is 1:1,000 to 5,000:1, and more preferably 1:100 to 1,000:1.

The molar concentration of the multivalent carboxylic acid compound in the mixed solution used for the manufacture of the metal complex is preferably 0.01 to 5.0 mol/L. Moreover, the molar concentration of the metal salt is preferably 0.01 to 5.0 mol/L, and the molar concentration of the organic ligand capable of multidentate binding is preferably 0.005 to 2.5 mol/L. If the molar concentration falls below this range upon the reaction, the yield of reaction undesirably decreases even though the target metal complex can still be obtained.

In the production method of the present invention, a monodentate organic ligand may be further added as long as the effect of the present invention is not impaired. The monodentate organic ligand refers to a neutral ligand having one site coordinated to the metal ion with a lone electron pair. The monodentate organic ligand is coordinated to the metal ion and incorporated in part of the metal complex, as with the organic ligand capable of multidentate binding. Examples of monodentate organic ligands include furan, thiophene, pyridine, quinoline, isoquinoline, acridine, trimethyl phosphine, triphenyl phosphine, triphenyl phosphite, methyl isocyanide, and the like. Among these, pyridine is preferable. The monodentate organic ligand may include a C₁₋₂₃ hydrocarbon group as a substituent. The monodentate organic ligand may be allowed to coexist from the beginning of the reaction, or may be added in the latter stage of the reaction.

When the metal complex obtained by the production method of the present invention contains the monodentate organic ligand, the proportion of the monodentate organic ligand is not particularly limited as long as the effect of the present invention is not impaired. For example, the composition ratio of the organic ligand capable of multidentate binding to the monodentate organic ligand is preferably 5:1 to 1,000:1, and more preferably 10:1 to 100:1, in terms of molar ratio. The composition ratio can be determined by analysis using, for example, gas chromatography, high-performance liquid chromatography, or NMR; however, the method is not limited thereto.

The average particle diameter of the resulting metal complex varies depending on the concentration during the manufacture, the amount of the conjugate acid of the counter anion of the metal ion used, and the like. The average particle diameter is preferably 0.1 to 10 µm, and more preferably 0.5 to 8 µm. When the average particle diameter is less than 0.1 µm, pressure loss increases when the metal complex is used by placing it in a column, thus making handling difficult. When the average particle diameter is greater than 10 µm, crystals are deteriorated during handling of the metal complex, and adsorption performance is reduced.

The particle size of the metal complex can be confirmed by using a laser diffraction method, a dynamic light scattering method, an imaging method, a settling method, or the like; however, the method is not limited thereto.

In the manufacture of the metal complex, the yield per hour of reaction time per liter of the solvent is calculated, and the resulting value is defined as productivity [g/h·L]. The productivity of the method for producing a metal complex according to the present invention is generally 0.05 g/h·L or more, preferably 0.1 g/h·L or more, and more preferably 1.0 g/h·L or more.

The metal complex obtained by the production method of the present invention has a one-dimensional, two-dimensional, or three-dimensional framework, depending on the type of multivalent carboxylic acid compound, metal ion, and organic ligand capable of multidentate binding to the metal ion to be used.

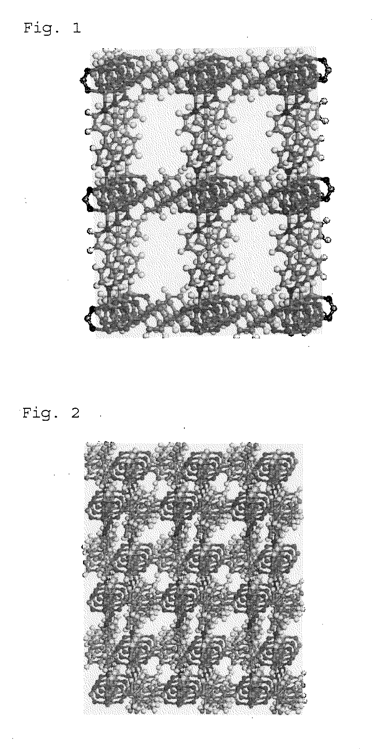

One detailed example is a metal complex comprising terephthalic acid as the multivalent carboxylic acid compound, zinc ion as the metal ion, and 4,4'-bipyridyl as the organic ligand capable of multidentate binding. The metal complex has a three-dimensional structure composed of two interpenetrated jungle-gym-type frameworks. The jungle-gym-type framework is structured such that 4,4'-bipyridyl is coordinated to the axial position of a metal ion in a paddle-wheel-type framework composed of a zinc ion and a carboxylate group of terephthalic acid. Fig. 1 is a schematic diagram illustrating a jungle-gym-type framework, and Fig. 2 is a schematic diagram illustrating a three-dimensional structure in which two jungle-gym-type frameworks are interpenetrated into each other.

The "jungle-gym-type framework" is defined as a jungle-gym-like three-dimensional structure in which an organic ligand capable of multidentate binding (e.g., 4,4'-bipyridyl) is coordinated to the axial position of a metal ion in a paddle-wheel-type framework composed of a metal ion and a multivalent carboxylic acid compound such as terephthalic acid, thus connecting the two-dimensional lattice sheets composed of the multivalent carboxylic acid compound and the metal ion. "A structure in which multiple jungle-gym-type frameworks are interpenetrated into each other" is defined as a three-dimensional framework in which multiple jungle-gym-type frameworks are interpenetrated into each other by filling each other's micropores.

For example, single-crystal X-ray structure analysis, powder X-ray crystal structure analysis, single-crystal neutron structure analysis, and powder neutron crystal structure analysis may be used to confirm whether the metal complex has the structure in which multiple jungle-gym-type frameworks are interpenetrated into each other; however, the method is not limited thereto.

Since the metal complex obtained by the production method of the present invention is a porous metal complex and can adsorb gas or the like in the micropores, it can be used as an adsorbent material, a storage material, and a separation material for various gases. However, the metal complex does not adsorb gas when a solvent is adsorbed. Accordingly, when the metal complex obtained by the production method of the present invention is used as an adsorbent material, storage material, or separation material, it is necessary to dry the metal complex under vacuum in advance to remove the solvent in the micropores. The vacuum drying may be generally performed at a temperature that does not decompose the metal complex (e.g., 298 K to 523 K or less); however, an even lower temperature (e.g., 298 K to 393 K or less) is preferable. This operation may be replaced by washing with supercritical carbon dioxide, which is more efficient.

The metal complex obtained by the production method of the present invention has excellent adsorption performance, storage performance, and separation performance with respect to various gases, such as carbon dioxide, hydrogen, carbon monoxide, oxygen, nitrogen, hydrocarbons having 1 to 4 carbon atoms (such as methane, ethane, ethylene, acetylene, propane, propene, methylacetylene, propadiene, butane, 1-butene, isobutene, 1-butyne, 2-butyne, 1,3-butadiene, and methylallene), noble gases (such as helium, neon, argon, krypton, and xenon), hydrogen sulfide, ammonia, sulfur oxides, nitrogen oxides, siloxanes (such as hexamethylcyclotrisiloxane and octamethylcyclotetrasiloxane), water vapor, and organic vapor (vaporizing gas of an organic substance that is in liquid form at ordinary temperature under ordinary pressure). Accordingly, the metal complex of the present invention is useful as an adsorbent material, a storage material, or a separation material for various gases, which are also within the technical scope of the present invention.

The term "organic vapor" means a vaporizing gas of an organic substance that is in liquid form at ordinary temperature under ordinary pressure. Examples of such organic substances include alcohols, such as methanol and ethanol; amines, such as trimethylamine; aldehydes, such as formaldehyde and acetaldehyde; hydrocarbons having 5 to 16 carbon atoms, such as pentane, isoprene, hexane, cyclohexane, heptane, methylcyclohexane, octane, 1-octene, cyclooctane, cyclooctene, 1,5-cyclooctadiene, 4-vinyl-1-cyclohexene, and 1,5,9-cyclododecatriene; aromatic hydrocarbons, such as benzene and toluene; ketones, such as acetone and methyl ethyl ketone; esters, such as methyl acetate and ethyl acetate; and halogenated hydrocarbons, such as methyl chloride and chloroform.

### Examples

The present invention will hereinafter be described specifically by using Examples. It should be noted, however, that the invention is not limited by these Examples. The analysis and evaluation in the following Examples and Comparative Examples were conducted as described below.

### (1) Calculation of Conversion of Raw Material Metal Salt by Pack Test

Using the Pack Test (produced by Kyoritsu Chemical-Check Lab., Corp.), the amount of metal ion dissolved in a solvent was quantified, and the conversion was calculated.

When the metal ion was copper ion: Pack Test Copper WAK-Cu

### (2) Measurement of Powder X-Ray Diffraction Pattern

The powder X-ray diffraction pattern was measured using an X-ray diffractometer based on the symmetric reflection method while scanning at a scanning rate of 1°/min within a diffraction angle (2θ) range of 5 to 50°. Details of the analysis conditions are shown below.
- Analysis Conditions
   Apparatus: Smart Lab, produced by Rigaku Corporation
   X-ray source: CuKα (λ = 1.5418 Å) 45 kV 200 mA
   Goniometer: Vertical Goniometer
   Detector: D/teX Ultra
   Step width: 0.02°
   Slit:
      Divergence slit = 2/3°
      Receiving slit = 0.3 mm
      Scattering slit = 2/3°

### (3) Measurement of Average Particle Diameter

The average particle diameter of a sample obtained by dispersing 10 mg of metal complex in 180 mL of methanol using ultrasonic waves was measured by a laser diffraction/scattering particle size distribution measuring apparatus. Analysis was performed on the assumption that the particles had a spherical shape. Details of the analysis conditions are shown below.
- Analysis Conditions
   Apparatus: Partica LA-950V2, produced by Horiba, Ltd.
   Light source: Semiconductor laser (650 nm), LED (405 nm)
   Measurement method: Flow cell measurement
   Temperature: 298 K
   Refractive index of metal complex: 2.00
   Refractive index of methanol: 1.33

### (4) Quantification of Monocarboxylic Acid Compound

The metal complex was dissolved in a deuterated solvent to form a homogeneous solution. Deuterated trifluoroacetic acid was added thereto, and the white precipitate was filtered to obtain a sample. The sample was subjected to ¹H-NMR measurement, and the monocarboxylic acid compound was quantified from the ratio of integral of the resulting spectrum. Details of the analysis conditions are shown below.
- Analysis Conditions
   Apparatus: JNM-LA500, produced by JEOL Ltd.
   Resonance frequency: 500 MHz
   Standard substance: Sodium 3-(trimethylsilyl)propanoate-d₄ Temperature: 298 K
   Flip angle: 30°
   Pulse repetition time: 7.0 s
   Integration number: 2,048 times

### (5) Measurement of Adsorption and Desorption Isotherms

The amounts of gas adsorbed and desorbed were measured according to the volumetric method by using a high-pressure gas adsorption measuring instrument to plot adsorption and desorption isotherms (in accordance with JIS Z8831-2). Before the measurement, the sample was dried at 373 K and 0.5 Pa for 5 hours to remove adsorbed water and the like. Details of the analysis conditions are shown below.
- Analysis Conditions
   Apparatus: BELSORP-HP, produced by Bel Japan, Inc.
   Equilibrium waiting time: 500 s

### (6) Calculation of Productivity

The productivity [g/L·h] of the metal complex was calculated as the yield per hour of reaction time per liter of the solvent.

### Example 1

### First Step

Under nitrogen atmosphere, 18.2 g (109 mmol, pKₐₗ = 3.51) of terephthalic acid, 21.8 g (109 mmol; pKₐₗ of acetic acid (counter anion) = 4.82, which was larger by 1.31 than pKₐₗ of terephthalic acid) of copper acetate monohydrate, and 26.2 g (436 mmol) of acetic acid were dispersed in 200 mL of methanol, and the mixture was stirred at 333 K in a suspended state. It was confirmed that the conversion of the raw material metal salt at the time of 21 hours after the start of the reaction calculated by using the Pack Test was 99%. Stirring was stopped 24 hours after the start of the reaction. After collecting the precipitated metal complex by suction filtration, the metal complex was washed three times with methanol to isolate an intermediate.

### Second Step

The isolated intermediate was dispersed in 133 mL of methanol under nitrogen atmosphere, and 8.54 g (54.7 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the resultant was dried at 373 K and 50 Pa for 8 hours, thereby obtaining 29.5 g of the target metal complex.

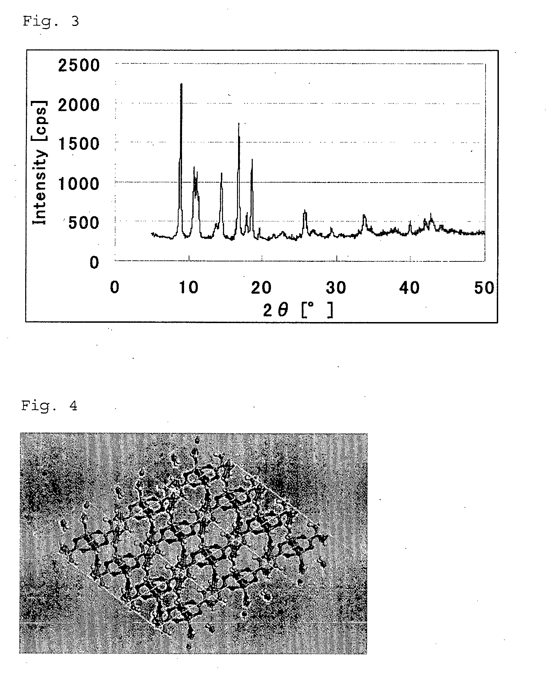

Fig. 3 shows the powder X-ray diffraction pattern of the resulting metal complex. The results of the powder X-ray crystal structure analysis revealed that the resulting metal complex had a structure in which two jungle-gym-type frameworks were interpenetrated into each other. The powder X-ray crystal structure analysis results are shown below. Further, Fig. 4 shows the crystal structure.
Triclinic (P-1)
a = 7.87355 Å
b = 8.94070 Å
c = 10.79101 Å
α = 67.14528°
β = 80.73986°
γ = 79.31579°
R_{wp} = 2.30%
R_{I} = 4.96%

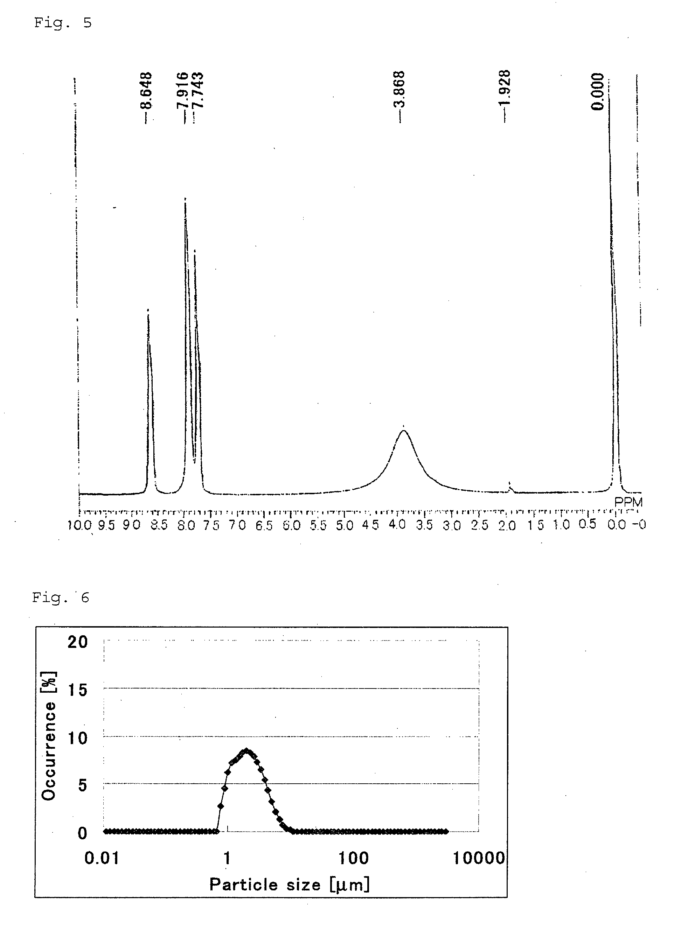

The resulting metal complex (10 mg) was dissolved in 700 mg of deuterated ammonia water (containing 0.4 wt% sodium 3-(trimethylsilyl)propanoate-d₄ as a standard substance) to perform ¹H-NMR measurement. Fig. 5 shows the resulting spectrum. The results of spectrum analysis revealed that the peak integral value attributed to protons at position 1 of the acetic acid at 1.927 ppm (s, 4H) was 30.080 when the peak integral value attributed to protons at positions 2, 3, 5, and 6 of the terephthalic acid at 7.915 ppm (s, 4H) was taken as 1,000. This indicated that the molar ratio of the terephthalic acid to acetic acid (terephthalic acid:acetic acid) contained in the metal complex was 25:1. In Fig. 6, the broad signal around 3.9 ppm is attributed to water.

The results of the powder X-ray crystal structure analysis and ¹H-NMR measurement revealed that the composition formula of the resulting metal complex was [Cu₂(C₈H₄O₄)₂₋ₓ(C₁₀H₈N₂)(CH₃COO)ₓ]ₘ (x = 0.08). n is a positive integer. Since the value x was small, the theoretical yield was calculated based on the molecular weight of the compound represented by [Cu₂(C₈H₄O₄)₂(C₁₀H₈N₂)]ₙ (copper:terephthalic acid:4,4'-bipyridyl = 2:2:1). Consequently, the yield of the resulting metal complex was 88%. Table 1 shows the results.

The productivity of the method for producing the metal complex was 3.28 g/L·h. Table 1 shows the results.

Fig. 6 shows the particle size distribution of the resulting metal complex. The results of the particle size distribution measurement revealed that the average particle diameter of the resulting metal complex was 2.27 µm. Table 1 shows the results.

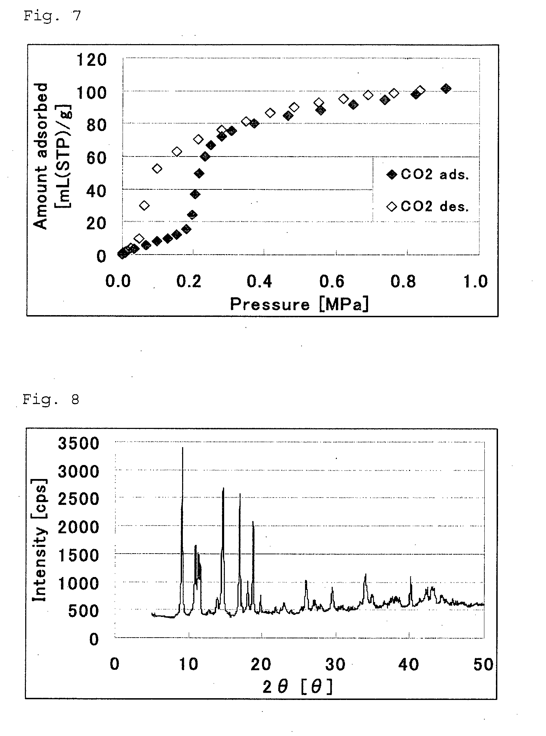

Adsorption and desorption isotherms of carbon dioxide on the resulting metal complex at 293 K were measured. Fig. 7 shows the adsorption and desorption isotherms.

### Example 2

### First Step

Under nitrogen atmosphere, 18.2 g (109 mmol, pKₐₗ = 3.51) of terephthalic acid, 24.7 g (109 mmol; pKₐₗ of formic acid (counter anion) = 3.75, which was larger by 0.24 than pKₐₗ of terephthalic acid) of copper formate tetrahydrate, and 5.04 g (109 mmol) of formic acid were dispersed in 200 mL of methanol, and the mixture was stirred at 333 K in a suspended state. It was confirmed that the conversion of the raw material metal salt at the time of 18 hours after the start of the reaction calculated by using the Pack Test was 99%. Stirring was stopped 24 hours after the start of the reaction. After collecting the precipitated metal complex by suction filtration, the metal complex was washed three times with methanol to isolate an intermediate.

### Second Step

The isolated intermediate was dispersed in 133 mL of methanol under nitrogen atmosphere, and 8.54 g (54.7 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the resultant was dried at 373 K and 50 Pa for 8 hours, thereby obtaining 29.3 g of the target metal complex.

Fig. 8 shows the powder X-ray diffraction pattern of the resulting metal complex. The results of the powder X-ray crystal structure analysis revealed that the resulting metal complex had a structure in which two jungle-gym-type frameworks were interpenetrated into each other, as with the metal complex obtained in Synthesis Example 1.

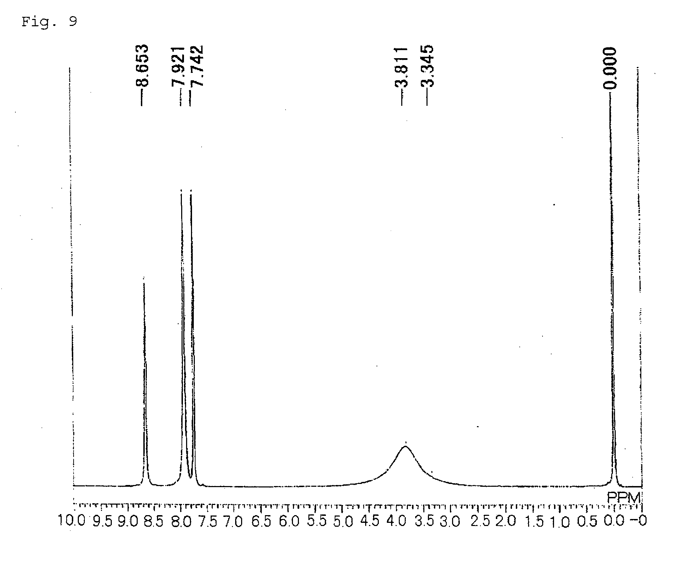

The resulting metal complex (10 mg) was dissolved in 700 mg of deuterated ammonia water (containing 0.4 wt% sodium 3-(trimethylsilyl)propanoate-d₄ as a standard substance) to perform ¹H-NMR measurement. Fig. 9 shows the resulting spectrum. The results of spectrum analysis revealed that the peak integral value attributed to protons at positions 2, 6, 2', and 6' of the 4,4'-bipyridyl at 8.653 ppm (s, 4H) was 501.1 when the peak integral value attributed to protons at positions 2, 3, 5, and 6 of the terephthalic acid at 7.921 ppm (s, 4H) was taken as 1,000. This indicated that the molar ratio of the terephthalic acid to 4,4'-bipyridyl (terephthalic acid:4,4'-bipyridyl) contained in the metal complex was 2:1. In Fig. 9, the broad signal around 3.8 ppm is attributed to water.

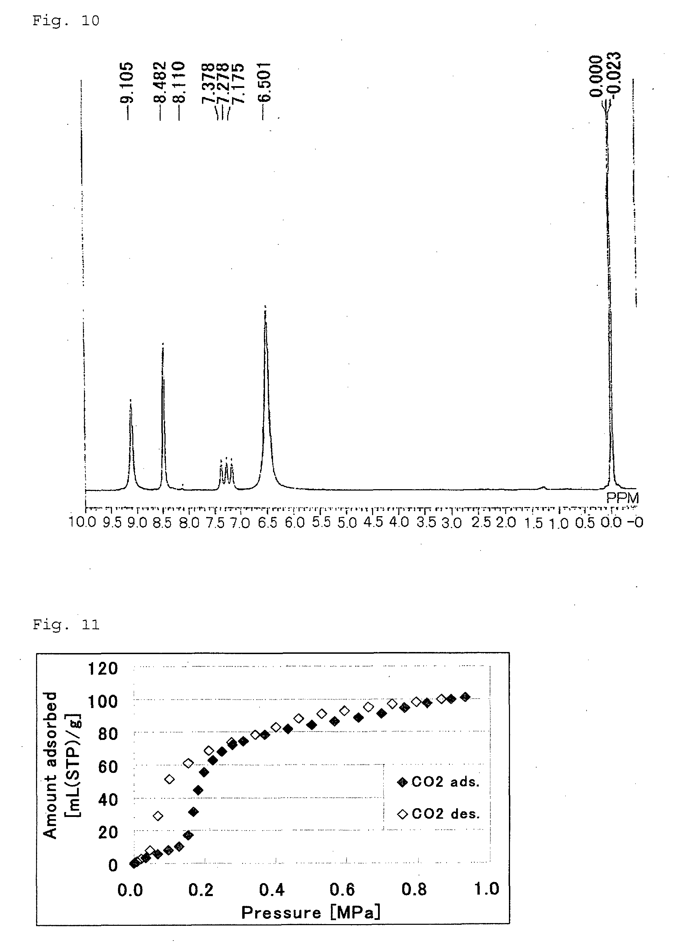

The resulting metal complex (10 mg) was dissolved in 700 mg of deuterated ammonia water, and 1,100 mg of deuterated trifluoroacetic acid was added thereto. After removing the produced white precipitate by filtration, 1 mg of sodium 3-(trimethylsilyl)propanoate-d₄ was added as a standard substance to the filtrate to perform ¹H-NMR measurement. Fig. 10 shows the resulting spectrum. The results of spectrum analysis revealed that the peak integral value attributed to protons of the formic acid at 8.129 ppm (s, 1H) was 5.228 when the sum of the peak integral value attributed to protons at positions 2, 6, 2', and 6' of the 4,4'-bipyridyl at 9.105 ppm (s, 4H) and the peak integral value attributed to protons at positions 3, 5, 3', and 5' of the 4,4'-bipyridyl at 8.482 ppm (s, 4H) was taken as 1,000. This indicated that the molar ratio of the 4,4'-bipyridyl to formic acid (4,4'-bipyridyl:formic acid) contained in the metal complex was 24:1.

Based on the above results, the molar ratio of the terephthalic acid to formic acid (terephthalic acid:formic acid) contained in the metal complex was calculated to be 48:1.

The yield of the resulting metal complex calculated as in Example 1 was 88%. Table 1 shows the results.

Moreover, the productivity in the manufacture of the resulting metal complex was 3.26 g/h·L. Table 1 shows the results.

The results of measuring the particle size distribution as in Example 1 revealed that the average particle diameter of the resulting metal complex was 3.48 µm. Table 1 shows the results.

Adsorption and desorption isotherms of carbon dioxide on the resulting metal complex at 293 K were measured. Fig. 11 shows the adsorption and desorption isotherms.

### Example 3

### First Step

Under nitrogen atmosphere, 9.09 g (54.7 mmol, pKₐₗ = 3.51) of terephthalic acid and 12.3 g (54.7 mmol; pKₐₗ of formic acid (counter anion) = 3.75, which was larger by 0.24 than pKₐₗ of terephthalic acid) of copper formate tetrahydrate were dispersed in 100 mL of methanol, and the mixture was stirred at 333 K in a suspended state. It was confirmed that the conversion of the raw material metal salt at the time of 21 hours after the start of the reaction calculated by using the Pack Test was 99%. Stirring was stopped 24 hours after the start of the reaction. After collecting the precipitated metal complex by suction filtration, the metal complex was washed three times with methanol to isolate an intermediate.

### Second Step

The isolated intermediate was dispersed in 67 mL of methanol under nitrogen atmosphere, and 4.27 g (27.4 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the resultant was dried at 373 K and 50 Pa for 8 hours, thereby obtaining 12.2 g of the target metal complex.

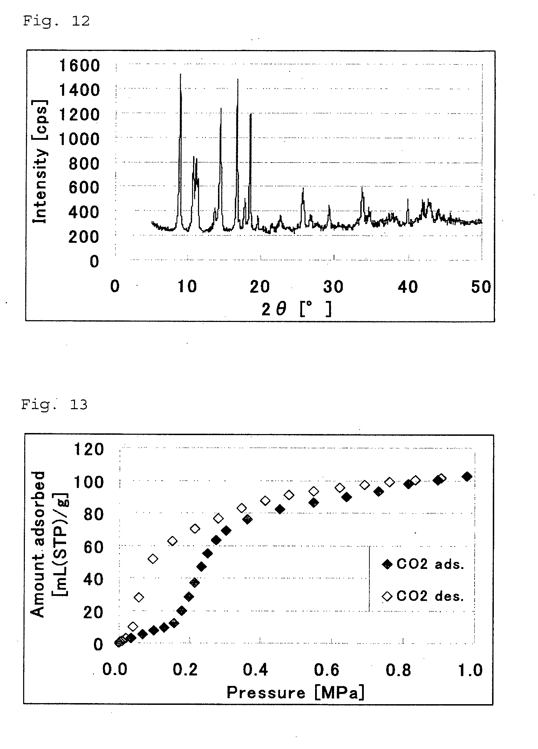

Fig. 12 shows the powder X-ray diffraction pattern of the resulting metal complex. The results of the powder X-ray crystal structure analysis revealed that the resulting metal complex had a structure in which two jungle-gym-type frameworks were interpenetrated into each other, as with the metal complex obtained in Synthesis Example 1.

The results of ¹H-NMR measurement performed as in Example 2 revealed that the molar ratio of the terephthalic acid to 4,4'-bipyridyl (terephthalic acid:4,4'-bipyridyl) contained in the metal complex was 2:1.

The results of ¹H-NMR measurement performed as in Example 2 revealed that the molar ratio of the 4,4'-bipyridyl to formic acid (4,4'-bipyridyl:formic acid) contained in the metal complex was 23:1.

Based on the above results, the molar ratio of the terephthalic acid to formic acid (terephthalic acid:formic acid) contained in the metal complex was calculated to be 46:1.

The yield of the resulting metal complex calculated as in Example 2 was 73%. Table 1 shows the results.

The productivity in the manufacture of the metal complex calculated as in Example 1 was 2.71 g/h·L. Table 1 shows the results.

The results of measuring the particle size distribution as in Example 1 revealed that the average particle diameter of the resulting metal complex was 1.25 µm. Table 1 shows the results.

Adsorption and desorption isotherms of carbon dioxide on the resulting metal complex at 293 K were measured. Fig. 13 shows the adsorption and desorption isotherms.

### Comparative Example 1

### First Step

Under nitrogen atmosphere, 3.90 g (23.5 mmol, pKₐₗ = 3.51) of terephthalic acid, 5.86 g (23.5 mmol; pKₐₗ of sulfuric acid (counter anion) = -5.00, which was smaller by 8.51 than pKₐₗ of terephthalic acid) of copper sulfate pentahydrate, and 42.3 g (704 mmol) of acetic acid were dissolved in 3,750 mL of methanol, and the mixture was stirred at 313 K. The conversion of the raw material metal salt at the time of 3 hours after the start of the reaction calculated by using the Pack Test was 40%. It was also confirmed that the conversion of the raw material metal salt at the time of 20 hours after the start of the reaction was 40%. Stirring was stopped 24 hours after the start of the reaction. After collecting the precipitated metal complex by suction filtration, the metal complex was washed three times with methanol to isolate an intermediate.

### Second Step

The isolated intermediate was dispersed in 2,000 mL of methanol under nitrogen atmosphere, and 1.83 g (11.7 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the resultant was dried at 373 K and 50 Pa for 8 hours, thereby obtaining 1.55 g of the target metal complex.

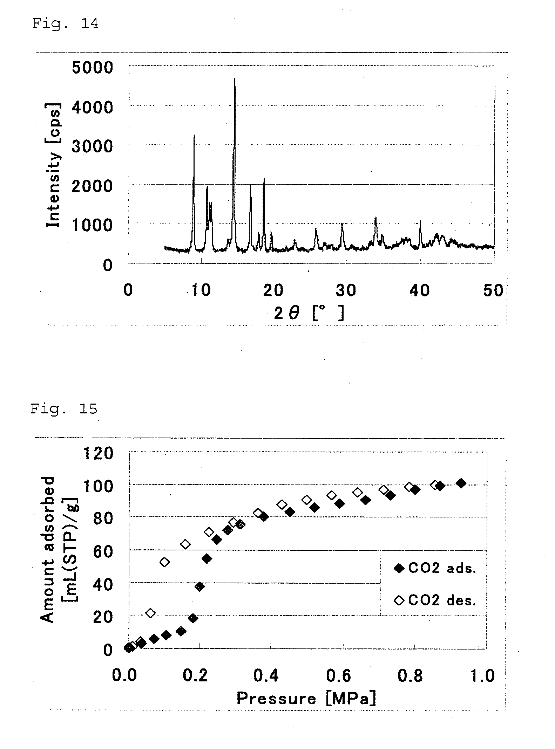

Fig. 14 shows the powder X-ray diffraction pattern of the resulting metal complex. The results of the powder X-ray crystal structure analysis revealed that the resulting metal complex had a structure in which two jungle-gym-type frameworks were interpenetrated into each other, as with the metal complex obtained in Synthesis Example 1.

The results of ¹H-NMR measurement performed as in Example 1 revealed that the molar ratio of the terephthalic acid to acetic acid (terephthalic acid:acetic acid) contained in the metal complex was 208:1.

The yield of the resulting metal complex calculated as in Example 1 was 22%. Table 1 shows the results.

The productivity in the manufacture of the resulting metal complex calculated as in Example 1 was 0.01 g/h·L. Table 1 shows the results.

The results of measuring the particle size distribution as in Example 1 revealed that the average particle diameter of the resulting metal complex was 4.77 µm. Table 1 shows the results.

Adsorption and desorption isotherms of carbon dioxide on the resulting metal complex at 293 K were measured. Fig. 15 shows the adsorption and desorption isotherms.

### Comparative Example 2

### First Step

Under nitrogen atmosphere, 3.90 g (23.5 mmol) of terephthalic acid, 5.86 g (23.5 mmol) of copper sulfate pentahydrate, and 32.4 g (704 mmol) of formic acid were dispersed in 3,750 mL of methanol, and the mixture was stirred at 313 K. The conversion of the raw material metal salt at the time of 3 hours after the start of the reaction calculated by using the Pack Test was 35%. It was also confirmed that the conversion of the raw material metal salt at the time of 24 hours after the start of the reaction was 37%. Stirring was stopped 24 hours after the start of the reaction. After collecting the precipitated metal complex by suction filtration, the metal complex was washed three times with methanol to isolate an intermediate.

### Second Step

The isolated intermediate was dispersed in 2,000 mL of methanol under nitrogen atmosphere, and 1.83 g (11.7 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the resultant was dried at 373 K and 50 Pa for 8 hours, thereby obtaining 1.68 g of the target metal complex.

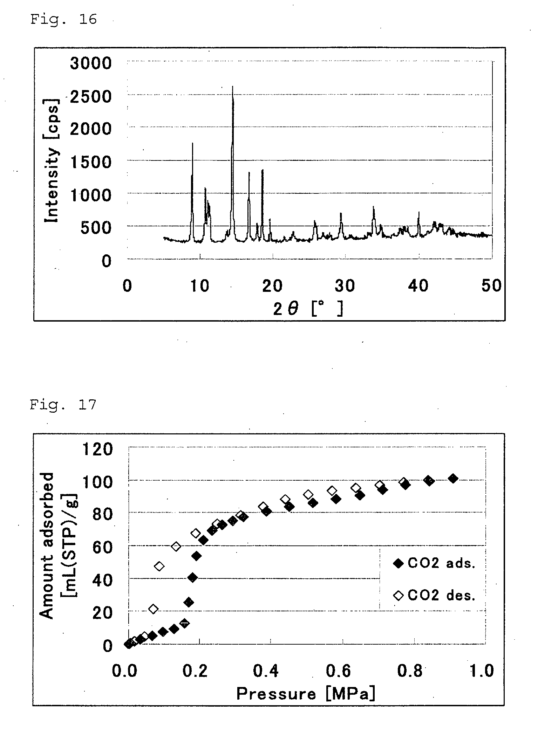

Fig. 16 shows the powder X-ray diffraction pattern of the resulting metal complex. The results of the powder X-ray crystal structure analysis revealed that the resulting metal complex had a structure in which two jungle-gym-type frameworks were interpenetrated into each other, as with the metal complex obtained in Synthesis Example 1.

The results of ¹H-NMR measurement performed as in Example 2 revealed that the molar ratio of the terephthalic acid to 4,4'-bipyridyl (terephthalic acid:4,4'-bipyridyl) contained in the metal complex was 2:1.

The results of ¹H-NMR measurement performed as in Example 2 revealed that the molar ratio of the 4,4'-bipyridyl to formic acid (4,4'-bipyridyl:formic acid) contained in the metal complex was 34:1.

Based on the above results, the molar ratio of the terephthalic acid to formic acid (terephthalic acid:formic acid) contained in the metal complex was calculated to be 68:1.

The yield of the resulting metal complex calculated as in Example 2 was 23%. Table 1 shows the results.

The productivity in the manufacture of the resulting metal complex calculated as in Example 1 was 0.01 g/h·L. Table 1 shows the results.

The results of measuring the particle size distribution as in Example 1 revealed that the average particle diameter of the resulting metal complex was 5.28 µm. Table 1 shows the results.

Adsorption and desorption isotherms of carbon dioxide on the resulting metal complex at 293 K were measured. Fig. 17 shows the adsorption and desorption isotherms.

### Comparative Example 3

Under nitrogen atmosphere, 1.50 g (9.00 mmol) of terephthalic acid, 1.80 g (9.00 mmol) of copper acetate monohydrate, and 25.8 g (450 mmol) of acetic acid were dissolved in 2,000 mL of methanol, and the mixture was stirred at 298 K in a solution state. It was confirmed that the conversion of the raw material metal salt at the time of 66 hours after the start of the reaction calculated by using the Pack Test was 94%. Further, 0.704 g (4.50 mmol) of 4,4'-bipyridyl was added 72 hours after the start of the reaction, and the mixture was stirred at 298 K for 48 hours. After collecting the precipitated metal complex by suction filtration, the metal complex was washed three times with methanol. Subsequently, the resultant was dried at 373 K and 50 Pa for 8 hours, thereby obtaining 2.46 g of the target metal complex.

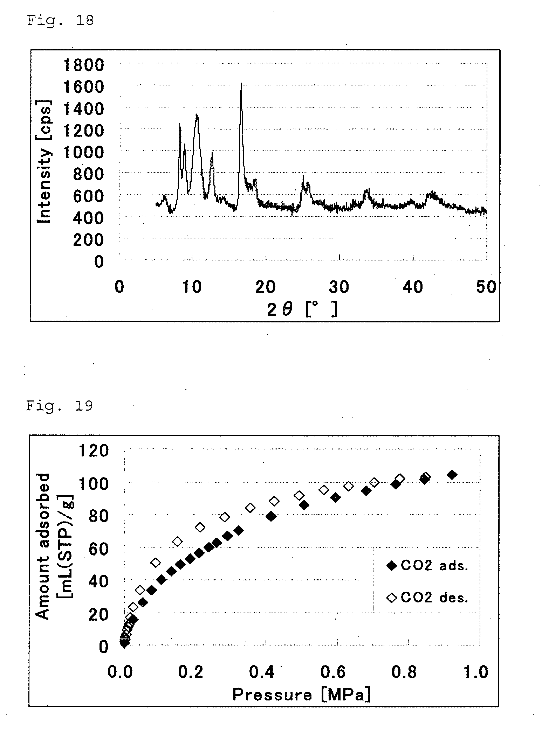

Fig. 18 shows the powder X-ray diffraction pattern of the resulting metal complex. The results of the powder X-ray crystal structure analysis revealed that the resulting metal complex had a structure in which two jungle-gym-type frameworks were interpenetrated into each other, as with the metal complex obtained in Synthesis Example 1.

The results of ¹H-NMR measurement performed as in Example 1 revealed that the molar ratio of the terephthalic acid to acetic acid (terephthalic acid:acetic acid) contained in the metal complex was 16:1.

The yield of the resulting metal complex calculated as in Example 1 was 88%. Table 1 shows the results.

The productivity in the manufacture of the resulting metal complex calculated as in Example 1 was 0.01 g/h·L. Table 1 shows the results.

The results of measuring the particle size distribution as in Example 1 revealed that the average particle diameter of the resulting metal complex was 0.08 µm. Table 1 shows the results.

Adsorption and desorption isotherms of carbon dioxide on the resulting metal complex at 293 K were measured. Fig. 19 shows the adsorption and desorption isotherms.

### Comparative Example 4

Under nitrogen atmosphere, 18.2 g (109 mmol, pKₐₗ = 3.51) of terephthalic acid, 21.8 g (109 mmol; pKₐₗ of acetic acid (counter anion) = 4.82, which was larger by 1.31 than pKₐₗ of terephthalic acid) of copper acetate monohydrate, and 26.2 g (434 mmol) of acetic acid were dispersed in 200 mL of methanol, and the mixture was stirred at 298 K in a suspended state. An attempt was made to calculate the conversion of the raw material metal salt at the time of 70 hours after the start of the reaction by using the Pack Test; however, the remaining amount was significantly more than the saturated solubility, and the conversion was thus not determined.

**Table 1**

| | Monocarboxylic acid | Productivity [g/h·L] | Average particle diameter [µm] |
|---|---|---|---|
| Example 1 | Acetic acid added | 3.28 | 2.27 |
| Example 2 | Formic acid added | 3.26 | 3.48 |
| Example 3 | Counter anion of copper formate | 2.71 | 1.25 |
| Comparative Example 1 | Acetic acid added | 0.01 | 4.77 |
| Comparative Example 2 | Formic acid added | 0.01 | 5.28 |
| Comparative Example 3 | Acetic acid added | 0.01 | 0.08 |

Moreover, in Example 1 and Comparative Example 3, the difference between the amount adsorbed at 0.3 MPa in the adsorption isotherm and the amount adsorbed at 0.1 MPa in the desorption isotherm was calculated, and the effective storage amount [mL(STP)/g] at 0.1 to 0.3 MPa was calculated. Table 2 shows the results.

**Table 2**

| | Effective storage amount [mL(STP)/g] |
|---|---|
| Example 1 | 24 |
| Comparative Example 3 | 12 |

According to Examples 1 to 3, the production method of the present invention can produce metal complexes having high gas adsorption performance at a high yield of 80% or more. On the other hand, in Comparative Examples 1 and 2, in which strong acid with a first dissociation exponent largely different from that of the multivalent carboxylic acid compound constituting the metal complex was used as the counter anion of the metal salt, the conversion of the raw material metal salt was low; thus, the yield and productivity of the resulting metal complexes were low. Moreover, in the production method of Comparative Example 3, which was performed in a dissolved state, rather than a suspended state, in the manufacture process, the yield of the resulting metal complex is high; however, it is evident that productivity is low and volume efficiency is poor. Furthermore, in the production method of Comparative Example 4, which was performed in a suspended state while lowering the temperature to 298 K, the conversion of the raw material metal salt was low, and thus an intermediate could not be collected. In addition, a comparison between the results of Example 1 and Comparative Example 3 in Tables 1 and 2 indicates that the metal complex obtained by the production method of the present invention has a larger average particle diameter and a higher effective gas storage amount. These results clearly indicate that the production method of the present invention is superior as the method for producing a metal complex having excellent gas adsorption, storage, and separation performance.

### Example 4

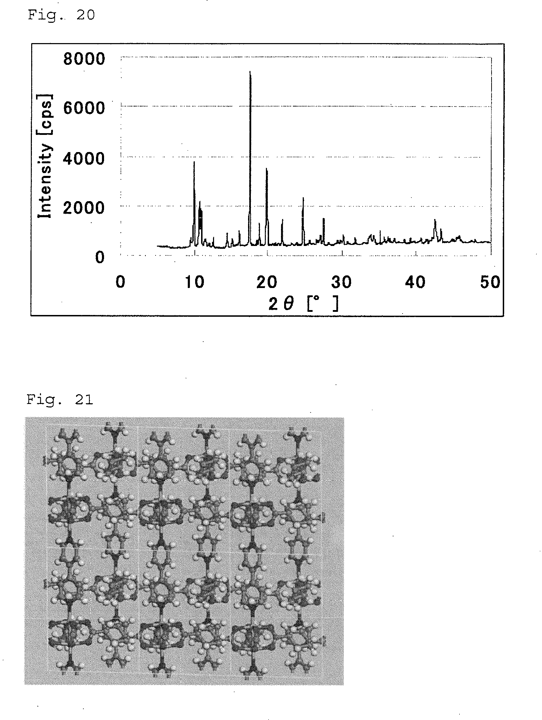

Under nitrogen atmosphere, 2.48 g (16.3 mmol, pKₐₗ = 4.18) of trans-1,4-cyclohexanedicarboxylic acid, 1.75 g (8.76 mmol; pKₐₗ of acetic acid (counter anion) = 4.82, which was larger by 0.64 than pKₐₗ of trans-1,4-cyclohexanedicarboxylic acid) of copper acetate monohydrate, and 0.760 g (4.85 mmol) of 4,4'-bipyridyl were dispersed in 600 mL of water, and the mixture was stirred at 353 K in a suspended state. It was confirmed that the conversion of the raw material metal salt at the time of 124 hours after the start of the reaction calculated by using the Pack Test was 96%. It was also confirmed that the conversion of the raw material metal salt at the time of 143 hours after the start of the reaction was 96%. Stirring was stopped 144 hours after the start of the reaction. Fig. 20 shows the powder X-ray diffraction pattern of the precipitated metal complex. A comparison with a simulation pattern determined from the structure analysis results of separately synthesized monocrystal revealed that the resulting metal complex had a structure in which two jungle-gym-type frameworks were interpenetrated into each other. The single crystal structure analysis results are shown below. Further, Fig. 21 shows the crystal structure.
Triclinic (P-1)
a = 10.804 (2) Å
b = 10.741(3) Å
c = 14.050(4) Å
α = 87.357(11)°
β = 88.031 (12)°
γ = 68.716(16)°
V = 1,517.347 Å
R = 0.0825
R_{w} = 0.0825

After collecting the precipitated metal complex by suction filtration, the metal complex was washed three times with methanol. Subsequently, the resultant was dried at 373 K and 50 Pa for 8 hours, thereby obtaining 2.69 g of the target metal complex.

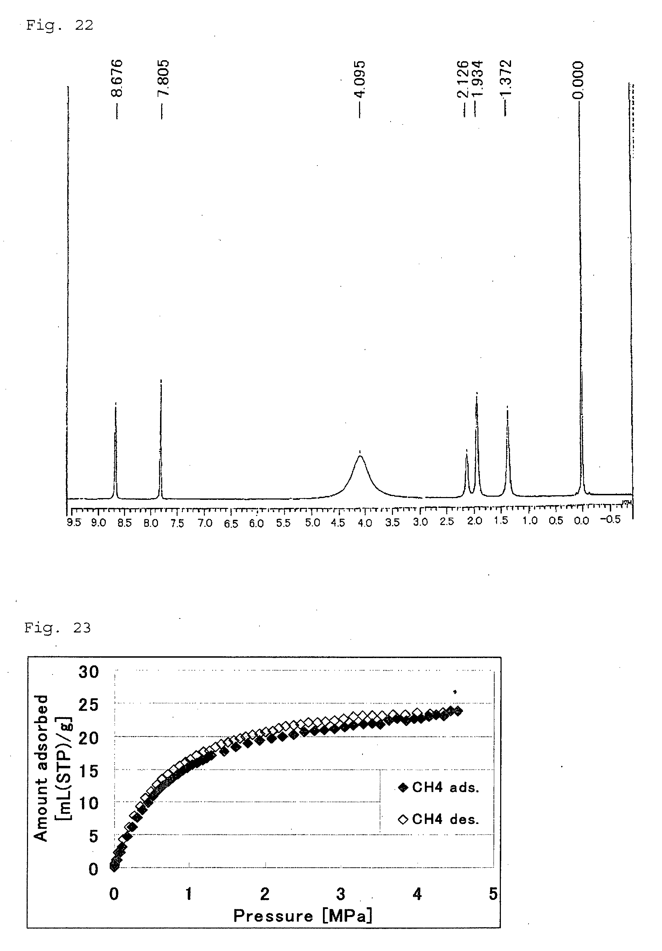

The resulting metal complex (10 mg) was dissolved in 800 mg of deuterated ammonia water (containing 0.4 wt% sodium 3-(trimethylsilyl)propanoate-d₄ as a standard substance) to perform ¹H-NMR measurement. Fig. 22 shows the resulting spectrum. The results of spectrum analysis revealed that the sum of the peak integral value attributed to the overlap of protons (s, 4H) at the axial position, among protons at positions 2, 3, 5, and 6 of the trans-1,4-cyclohexanedicarboxylic acid, with protons (s, 3H) at position 1 of the acetic acid at 1.934 ppm, and the peak integral value attributed to protons at positions 1 and 4 of the trans-1,4-cyclohexanedicarboxylic acid at 2.126 ppm (s, 2H) was 1,503 when the peak integral value attributed to protons at the equatorial position, among protons at positions, 2, 3, 5, and 6 of the trans-1,4-cyclohexanedicarboxylic acid, at 1.372 ppm (s,4H) was taken as 1,000. This indicated that the molar ratio of the trans-1,4-cyclohexanedicarboxylic acid to acetic acid (trans-1,4-cyclohexanedicarboxylic acid:acetic acid) contained in the metal complex was 250:1. In Fig. 22, the broad signal around 4.1 ppm is attributed to water.

The results of the powder X-ray crystal structure analysis and ¹H-NMR measurement revealed that the composition formula of the resulting metal complex was [Cu₂(C₈H₁₀O₄)₂₋ₓ(C₁₀H₈N₂) (CH₃COO)ₓ]ₘ (x = 0.08). n is a positive integer. Since the value x was small, the theoretical yield was calculated based on the molecular weight of the compound represented by [Cu₂(C₈H₁₀O₄)₂(C₁₀H₈N₂)]ₙ (copper:trans-1,4-cyclohexanedicarboxylic acid:4,4'-bipyridyl = 2:2:1). The results revealed that the yield of the resulting metal complex was 90%.

Adsorption and desorption isotherms of methane on the resulting metal complex at 298 K were measured. Fig. 23 shows the adsorption and desorption isotherms.

### Comparative Example 5

Under nitrogen atmosphere, 2.48 g (16.3 mmol, pKₐₗ = 4.18) of trans-1,4-cyclohexanedicarboxylic acid, 2.21 g (8.76 mmol; pKₐₗ of nitric acid (counter anion) = -1.40, which was smaller by 5.58 than pKₐₗ of trans-1,4-cyclohexanedicarboxylic acid) of copper nitrate hemi(pentahydrate), and 0.760 g (4.85 mmol) of 4,4'-bipyridyl were dispersed in 600 mL of water, and the mixture was stirred at 353 K in a suspended state. It was confirmed that the conversion of the raw material metal salt at the time of 26 hours after the start of the reaction calculated by using the Pack Test was 5%. It was also confirmed that the conversion of the raw material metal salt at the time of 153 hours after the start of the reaction was 5%. Stirring was stopped 154 hours after the start of the reaction.

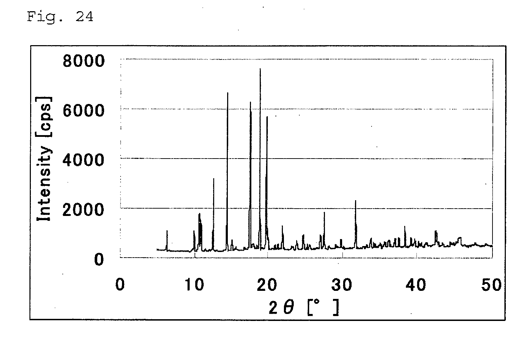

Fig. 24 shows the powder X-ray diffraction pattern of the precipitated metal complex. A comparison with a simulation pattern determined from the structure analysis results of separately synthesized monocrystal revealed that the resulting metal complex had a structure in which two jungle-gym-type frameworks were interpenetrated into each other, as with the metal complex obtained in Synthesis Example 4.

According to Example 4, the production method of the present invention can produce a metal complex having high gas adsorption performance at a high yield of 80% or more. On the other hand, in Comparative Example 5, in which a strong acid with a first dissociation exponent largely different from that of the multivalent carboxylic acid compound constituting the metal complex was used as the counter anion of the metal salt, the conversion of the raw material metal salt was low; thus, the yield and productivity of the resulting metal complex were low. These results clearly indicate that the production method of the present invention is superior as the method for producing a metal complex having excellent gas adsorption, storage, and separation performance.

### Industrial Applicability

The metal complex obtained by the production method of the present invention can be used as an adsorbent material for adsorbing various gases, a storage material for storing various gases, or a separation material for separating various gases.

## Claims

1. A method for producing a metal complex comprising a multivalent carboxylic acid compound, at least one metal ion selected from ions of metals belonging to Groups 2 to 13 of the periodic table, an organic ligand capable of multidentate binding to the metal ion, and a monocarboxylic acid compound;
the method comprising the step of reacting the multivalent carboxylic acid compound, the at least one metal ion selected from ions of metals belonging to Groups 2 to 13 of the periodic table, the organic ligand capable of multidentate binding to the metal ion, and the monocarboxylic acid compound in a single stage or multiple stages,
wherein in the reacting step, the metal ion is used in the form of a metal salt having a counter anion of the metal ion, a conjugate acid of the counter anion having a first dissociation exponent larger by 0 to 6 than that of the multivalent carboxylic acid compound, and
at least one of the multivalent carboxylic acid compound, the metal ion, the organic ligand capable of multidentate binding, and the monocarboxylic acid compound is reacted in a suspended state.

2. The method for producing a metal complex according to claim 1, wherein the reacting step is performed in a solvent.

3. The method for producing a metal complex according to claim 1 or 2, wherein the reacting step comprises:
a first step of reacting the multivalent carboxylic acid compound, the metal ion, and the monocarboxylic acid compound; and
a second step of reacting a product obtained in the first step with the organic ligand capable of multidentate binding.

4. The method for producing a metal complex according to claim 3, wherein the first step is performed at a reaction temperature of 303 to 373 K.

5. The method for producing a metal complex according to any one of claims 1 to 4, wherein the counter anion of the metal ion is an aliphatic monocarboxylate ion.

6. The method for producing a metal complex according to any one of claims 1 to 5, wherein the multivalent carboxylic acid compound is a dicarboxylic acid compound.

7. The method for producing a metal complex according to any one of claims 1 to 6, wherein the organic ligand capable of multidentate binding is an organic ligand capable of bidentate binding.

8. The method for producing a metal complex according to claim 7, wherein the organic ligand capable of bidentate binding belongs to the D_{∞h} point group and has a longitudinal length of 7.0 Å or more and 16.0 Å or less.

9. The method for producing a metal complex according to any one of claims 1 to 8, wherein the metal complex obtained by the method for producing a metal complex has an average particle diameter of 0.1 to 10 µm.

10. A metal complex obtained by the production method according to any one of claims 1 to 9.
